# EUROPÄISCHE PATENTSCHRIFT

(11) **EP 2 825 247 B1**
(45) Veröffentlichungstag und Bekanntmachung des Hinweises auf die Patenterteilung: **27.11.2019**
(21) Anmeldenummer: 13720224.8
(22) Anmeldetag: 14.03.2013
(51) Int. Cl.: A61M 39/10

(54) **MEDIZINISCHE VORRICHTUNG MIT EINER BUCHSEN-EINHEIT ZUM ANSCHLUSS EINER VORRICHTUNG ZUR BEREITSTELLUNG VON MEDIZINISCHEN FLÜSSIGKEITEN SOWIE VORRICHTUNG ZUR BEREITSTELLUNG VON MEDIZINISCHEN FLÜSSIGKEITEN MIT EINER STECKER-EINHEIT ZUM ANSCHLUSS AN EINE MEDIZINISCHE VORRICHTUNG**
MEDICAL DEVICE COMPRISING A SOCKET UNIT FOR CONNECTING A DEVICE FOR PROVIDING MEDICAL LIQUIDS AND DEVICE FOR PROVIDING MEDICAL LIQUIDS, COMPRISING A PLUG UNIT FOR CONNECTION TO A MEDICAL DEVICE
DISPOSITIF MÉDICAL DOTÉ D'UNE UNITÉ CONNECTEUR FEMELLE POUR LE RACCORDEMENT D'UN DISPOSITIF DE FOURNITURE DE LIQUIDES MÉDICINAUX ET DISPOSITIF DE FOURNITURE DE LIQUIDES MÉDICINAUX DOTÉ D'UNE UNITÉ CONNECTEUR MÂLE POUR LE RACCORDEMENT À UN DISPOSITIF MÉDICAL

(30) Priorität: 16.03.2012 DE 102012005187; 16.03.2012 US 201261611605 P
(43) Veröffentlichungstag der Anmeldung: 21.01.2015
(73) Patentinhaber: Fresenius Medical Care Deutschland GmbH, 61352 Bad Homburg (DE)
(72) Erfinder: BRANDL, Matthias, 97631 Bad Königshofen (DE); FAULHABER, Thomas, 97493 Bergrheinfeld (DE); HÖRMANN, Jörn, 66836 St. Ingbert (DE); KUGELMANN, Franz, 66606 St. Wendel/Bliesen (DE); ÖRTER, Gökhan, 35789 Weilmünster (DE); STERZER, Rafael, 97422 Schweinfurt (DE)
(74) Vertreter: Oppermann, Frank
(86) Internationale Anmeldenummer: PCT/EP2013/000769
(87) Internationale Veröffentlichungsnummer: WO 2013/135386

(56) Entgegenhaltungen:
- EP-A1- 2 130 565
- EP-A1- 2 452 720
- EP-A2- 0 575 970
- WO-A1-2010/140687

## Beschreibung

Die Erfindung betrifft eine medizinische Vorrichtung mit einer Buchsen-Einheit zum Anschluss einer Stecker-Einheit einer Vorrichtung zur Bereitstellung von medizinischen Flüssigkeiten, wobei die medizinische Vorrichtung insbesondere eine extrakorporale Blutbehandlungsvorrichtung, beispielsweise extrakorporale Dialysevorrichtung oder Vorrichtung zur Peritonealdialyse, oder eine Vorrichtung zum Befüllen der Vorrichtung zur Bereitstellung von medizinischen Flüssigkeiten ist. Darüber hinaus betrifft die Erfindung eine Anordnung mit einer medizinischen Vorrichtung und einer Vorrichtung zur Bereitstellung von medizinischen Flüssigkeiten.

Zum Anschluss von externen Komponenten an medizintechnische Einrichtungen ist eine Vielzahl von Konnektoren bekannt. Der Zugang zu den medizintechnischen Einrichtungen erfolgt im Allgemeinen mittels Steckern, die in passende Buchsen der medizintechnischen Einrichtungen eingesteckt werden. Insofern verfügen die medizintechnischen Einrichtungen, die nachfolgend als medizinische Vorrichtungen bezeichnet werden, über eine entsprechende Buchsen-Einheit, während die externen Komponenten eine Stecker-Einheit aufweisen.

Zur Behandlung von nierenkranken Patienten finden Blutbehandlungsvorrichtungen Verwendung, zu denen insbesondere die bekannten extrakorporalen Dialysevorrichtungen oder Vorrichtungen zur Peritonealdialyse gehören. Zur Blutreinigung des Patienten ist die Bereitstellung von medizinischen Behandlungsflüssigkeiten erforderlich. Zu diesen zählen beispielsweise die Dialysierflüssigkeit oder Substitutionsflüssigkeit. In der sogenannten Automatic Peritoneal Dialysis (APD) oder der Akutdialyse werden die medizinischen Behandlungsflüssigkeiten in den Blutbehandlungsvorrichtungen automatisch verarbeitet. Die Behandlungsflüssigkeiten werden in Flüssigkeitsreservoirs bereitgestellt, die an die Behandlungsvorrichtungen angeschlossen werden. Die frische Dialysierflüssigkeit wird aus dem Flüssigkeitsreservoir in die Blutbehandlungsvorrichtung und verbrauchte Flüssigkeit aus der Behandlungsvorrichtung in das Flüssigkeitsreservoir gepumpt. Das Flüssigkeitsreservoir kann bereits ein Konzentrat enthalten, das mit Wasser verdünnt wird. In diesem Fall muss das Flüssigkeitsreservoir nur mit Wasser befüllt werden. Daher wird in diesem Zusammenhang auch Wasser als medizinische Flüssigkeit verstanden. Es ist auch möglich, dass mehrere Flüssigkeitsreservoirs mit einer Blutbehandlungsvorrichtung verbunden sind, wenn in der Behandlungsvorrichtung eine gebrauchsfertige Behandlungsflüssigkeit durch Mischen mehrerer Flüssigkeiten hergestellt wird. Der Anschluss der Flüssigkeitsreservoirs an die Blutbehandlungsvorrichtungen erfolgt wieder mit einer Stecker-Einheit, die in eine Buchsen-Einheit der Blutbehandlungsvorrichtung eingesteckt wird.

Zum Befüllen der Vorrichtungen zur Bereitstellung von Dialyseflüssigkeit sind Vorrichtungen bekannt, an die sich die Vorrichtungen zur Bereitstellung von Dialysierflüssigkeit anschließen lassen. Dafür verfügen die Vorrichtungen zum Befüllen wiederum über eine Buchsen-Einheit, die sich mit der Stecker-Einheit der Vorrichtung zur Bereitstellung von Dialysierflüssigkeit konnektieren lässt.

Eine Vorrichtung zur Bereitstellung einer Behandlungsflüssigkeit ist beispielsweise aus der EP 0 575 970 A2 bekannt. Die bekannte Vorrichtung zur Bereitstellung von Dialysierflüssigkeit umfasst einen Beutel zur Aufnahme der Flüssigkeit, an dem eine Schlauchleitung angeschlossen ist, die an ihrem freien Ende mit einem Stecker verbunden ist. Die Dialysevorrichtung verfügt über eine Buchse, in die der Stecker eingesteckt wird. Mit dem Stecker und der Buchse können zwei Strömungsverbindungen hergestellt werden, um frische Dialysierflüssigkeit aus dem Beutel in die Dialysevorrichtung und verbrauchte Dialysierflüssigkeit zurück in den Beutel leiten zu können. Zur Sicherung des Steckers in der Buchse gegen Herausrutschen weist der Stecker Rastnasen auf, die in Ausnehmungen der Buchse greifen, wenn der Stecker vollständig in die Buchse eingesteckt ist.

Der Anschluss der Vorrichtung zur Bereitstellung von medizinischen Flüssigkeiten an die Blutbehandlungsvorrichtung oder die Vorrichtung zum Befüllen der Vorrichtung zur Bereitstellung von medizinischen Flüssigkeiten soll für das medizinische Personal möglichst einfach und sicher sein.

Die EP 2 130 565 A1 beschreibt eine Kupplung für ein medizinisches Instrument, die einen ersten und einen zweiten Kupplungsteil aufweist. Der erste Kupplungsteil ist eine Buchse, die über eine Schlauchleitung mit einer Spül- oder Saugquelle verbunden wird. Der zweite Kupplungsteil ist ein Stecker, der über eine Schlauchleitung mit einem Spül- oder Saug-instrument verbunden ist. Der Stecker weist ein Ansatzstück auf, das in ein Aufnahmestück der Buchse eingeschoben wird. Ansatzstück und Aufnahmestück verfügen über Verriegelungsmittel die derart ausgebildet sind, dass beim Einstecken des Steckers in die Buchse der Stecker in der Buchse automatisch verriegelt wird. Die Verrieglung des Steckers in die Buchse setzt voraus, dass der Stecker zur Betätigung der Verriegelungselemente von Hand vollständig in die Buchse eingeschoben wird.

Aus der WO 2010/140687 A1 ist eine verriegelbare Schraubkappe für eine Flasche bekannt, die mit der Hand auf die Flasche aufgeschraubt wird. Die EP 2 452 720 A1 beschreibt eine Kupplung mit zwei Kupplungsteilen, die jeweils einen Ventilkörper aufweisen.

Der Erfindung liegt die Aufgabe zu Grunde, die Versorgung der Blutbehandlungsvorrichtungen, insbesondere der extrakorporalen Dialysevorrichtungen oder Vorrichtungen zur Peritonealdialyse mit medizinischen Flüssigkeiten, insbesondere Dialysierflüssigkeit, für das medizinische Personal zu vereinfachen und die Sicherheit der Blutbehandlung zu erhöhen.

Die Lösung dieser Aufgabe erfolgt erfindungsgemäß mit den Merkmalen der unabhängigen Patentansprüche. Vorteilhafte Ausführungsformen der Erfindung sind Gegenstand der Unteransprüche.

Die erfindungsgemäße medizinische Blutbehandlungsvorrichtung oder die erfindungsgemäße Vorrichtung zur Befüllung der Vorrichtung zur Bereitstellung von medizinischen Flüssigkeiten verfügt über eine Buchsen-Einheit, während die erfindungsgemäße Vorrichtung zur Bereitstellung von medizinischen Flüssigkeiten über eine Stecker-Einheit verfügt.

Die Buchsen-Einheit und die Stecker-Einheit zeichnen sich dadurch aus, dass sich mit beiden Einheiten eine sichere Verbindung zwischen der Vorrichtung zur Bereitstellung von medizinischen Flüssigkeiten und einer Blutbehandlungsvorrichtung einerseits oder der Vorrichtung zum Befüllen der Vorrichtung zur Bereitstellung von medizinischen Flüssigkeiten andererseits einfach herstellen lässt.

Zur Herstellung der Strömungsverbindung verfügt die Buchsen-Einheit über mindestens ein Anschlussstück, während die Stecker-Einheit über mindestens einen Konnektor verfügt, wobei sich eine flüssigkeitsdichte Verbindung herstellen lässt, wenn das Anschlussstück an den Konnektor angeschlossen wird.

Bei einer bevorzugten Ausführungsform weist die Buchsen-Einheit ein erstes Anschlussstück zum Anschluss eines ersten Konnektors der Stecker-Einheit und ein zweites Anschlussstück zum Anschluss eines zweiten Konnektors der Stecker-Einheit auf, sodass eine erste Strömungsverbindung zum Zuführen frischer Behandlungsflüssigkeit und eine zweite Strömungsverbindung zum Abführen verbrauchter Behandlungsflüssigkeit hergestellt werden kann.

Für das Grundprinzip der Erfindung ist unerheblich, wie Anschlussstücke und Konnektoren ausgebildet sind. Anschlussstück und Konnektor können ihrerseits Stecker bzw. Buchsen sein. Das Grundprinzip der Erfindung liegt darin, dass die Buchsen-Einheit über eine Antriebseinheit aufweisende Mittel verfügt, mit denen entweder das mindestens eine Anschlussstück der Buchsen-Einheit relativ zu dem mindestens einen Konnektor der Stecker-Einheit oder der mindestens eine Konnektor der Stecker-Einheit relativ zu dem mindestens einen Anschlussstück der Buchsen-Einheit bewegt wird. Dadurch erfolgt eine automatische Konnektion von Anschlussstück und Konnektor. Es ist daher nicht erforderlich, die Stecker-Einheit vollständig in die Buchsen-Einheit einzustecken. Es genügt die Stecker-Einheit lose in die Buchsen-Einheit einzusetzen. Die flüssigkeitsdichte Konnektion von Anschlussstück und Konnektor erfolgt dann automatisch.

Die Buchsen-Einheit verfügt über Mittel zum Verbinden der Stecker-Einheit und der Buchsen-Einheit, die derart ausgebildet sind, dass bei der Ausführung der Relativbewegung zwischen dem mindestens einen Anschlussstück der Buchsen-Einheit und dem mindestens einen Konnektor der Stecker-Einheit die zunächst nur lose in die Buchsen-Einheit eingesetzte Stecker-Einheit fest mit der Buchsen-Einheit verbunden wird.

Bei einer bevorzugten Ausführungsform erfolgt die Verbindung von Stecker- und Buchsen-Einheit gleichzeitig mit der Ausführung der Relativbewegung. Es ist aber auch möglich, dass Stecker- und Buchsen-Einheit erst miteinander verbunden werden, bevor die Konnektion von Anschlussstück und Konnektor erfolgt.

Die Erfindung sieht zwei alternative Ausführungsformen vor, wobei die eine Ausführungsform vorzugsweise bei der Blutbehandlungsvorrichtung Verwendung findet, während die andere Ausführungsform vorzugsweise bei der Vorrichtung verwendet wird, mit der die Vorrichtung zur Bereitstellung von medizinischen Flüssigkeiten befüllt wird.

Bei der ersten alternativen Ausführungsform ist das mindestens eine Anschlussstück der Buchsen-Einheit unbeweglich an der Buchsen-Einheit angeordnet, wobei die Mittel zum Bewegen des mindestens einen Anschlussstücks der Buchsen-Einheit oder des mindestens einen Konnektors der Stecker-Einheit derart ausgebildet sind, dass die Stecker-Einheit auf die Buchsen-Einheit zu- oder von der Buchsen-Einheit wegbewegt wird. Bei dieser Ausführungsform findet also eine Relativbewegung zwischen Stecker-Einheit und Buchsen-Einheit statt, wobei die Stecker-Einheit automatisch in die Buchsen-Einheit gezogen wird, um Anschussstück und Konnektor zu konnektieren.

Die Mittel zum Verbinden der Stecker-Einheit und der Buchsen-Einheit weisen bei der ersten alternativen Ausführungsform ein in Längsrichtung bewegbares Aufnahmestück auf, in das ein komplementäres Ansatzstück der Stecker-Einheit passend eingesetzt werden kann. Die Mittel zum Bewegen des mindestens einen Anschlussstücks der Buchsen-Einheit oder des mindestens einen Konnektors der Stecker-Einheit weisen vorzugsweise einen elektromotorischen Antrieb auf, der das Aufnahmestück bewegt. Anstelle eines elektromotorischen Antriebs kann aber auch ein pneumatischer Antrieb vorgesehen sein.

Eine weitere bevorzugte Ausführungsform sieht vor, dass das Aufnahmestück der Buchsen-Einheit als ein in Längsrichtung verschiebbarer rohrförmiger Körper ausgebildet ist, in den das Ansatzstück der Stecker-Einheit einsetzbar ist.

Bei einer weiteren besonders bevorzugten Ausführungsform weist das Aufnahmestück der Buchsen-Einheit Ausnehmungen zur Aufnahme von Rastnasen des Ansatzstücks der Stecker-Einheit auf. Dadurch kann eine einrastende Verbindung hergestellt werden. Damit sind Aufnahmestück und Ansatzstück aber noch nicht verriegelt.

Zur Verriegelung des Ansatzstücks in dem Aufnahmestück weisen die Mittel zum Verbinden der Stecker-Einheit und der Buchsen-Einheit vorzugsweise einen stiftförmigen Körper auf, der in das Ansatzstück der Stecker-Einheit einführbar ist, sodass zur Herstellung einer festen Verbindung zwischen Stecker- und Buchsen-Einheit das Ansatzstück der Stecker-Einheit aufgespreizt wird. Damit ist das Ansatzstück der Stecker-Einheit in dem Aufnahmestück der Buchsen-Einheit gegen Herausrutschen gesichert. Die Verriegelung von Ansatzstück und Aufnahmestück erfolgt bei der besonders bevorzugten Ausführungsform dadurch, dass mit dem Aufspreizen des Ansatzstücks die Rastnasen in den Ausnehmungen gegen Herausrutschen gesichert werden.

Der stiftförmige Körper zum Aufspreizen des Ansatzstücks der Stecker-Einheit ist in der Buchsen-Einheit vorzugsweise unbeweglich und vorzugsweise in dem rohrförmigen Körper des Aufnahmestücks angeordnet. Daraus ergibt sich ein kompakter Aufbau.

Eine weitere besonders bevorzugte Ausführungsform der Erfindung sieht die automatische Erkennung des Ansatzstücks der Stecker-Einheit in dem Aufnahmestück der Buchsen-Einheit vor. Die Mittel zum Detektieren des Ansatzstücks in dem Aufnahmestück weisen vorzugsweise ein federnd vorgespanntes Tastelement auf, das derart in der Buchsen-Einheit angeordnet ist, dass das Tastelement beim Einführen des Ansatzstücks der Stecker-Einheit in das Aufnahmestück der Buchsen-Einheit entgegen der Federspannung verschoben wird. Die Verschiebung des Tastelements kann mit bekannten Mitteln erkannt werden. Beispielsweise können hierzu elektrische Kontakte, die beim Verschieben des Tastelements geschlossen bzw. geöffnet werden, oder eine Lichtschranke vorgesehen sein, mit der die Stellung des Tastelements detektiert wird.

Bei einer weiteren besonders bevorzugten Ausführungsform sind das Aufnahmestück, der röhrförmige Körper und der stiftförmige Körper der Buchsen-Einheit konzentrisch angeordnet, sodass sich ein besonders kompakter Aufbau ergibt.

Im Folgenden wird ein Ausführungsbeispiel der Erfindung unter Bezugnahme auf die Figuren näher erläutert.

Es zeigen:
- Fig. 1:: eine Vorrichtung zur Bereitstellung einer medizinischen Flüssigkeit, insbesondere Dialysierflüssigkeit, zusammen mit einer Blutbehandlungsvorrichtung und einer Vorrichtung zum Befüllen der Vorrichtung zur Bereitstellung von Dialysierflüssigkeit in stark vereinfachter schematischer Darstellung,
- Fig. 2: die Stecker-Einheit der Vorrichtung zur Bereitstellung von Dialysierflüssigkeit zusammen mit der Buchsen-Einheit der Blutbehandlungsvorrichtung von Fig. 1 in perspektivischer Darstellung,
- Fig. 3: die Stecker-Einheit und die Buchsen-Einheit von Fig. 2 in geschnittener Darstellung, wobei Stecker- und Buchsen-Einheit nicht miteinander verbunden sind,
- Fig. 4: die Stecker-Einheit und die Buchseneinheit von Fig. 2 in geschnittener Darstellung, wobei die Buchsen-Einheit zum Anschluss der Stecker-Einheit vorbereitet ist,
- Fig. 5: einen Schnitt durch die Stecker-Einheit und Buchsen-Einheit von Fig. 2, wobei die Stecker-Einheit in die Buchsen-Einheit lose eingesetzt ist, und
- Fig. 6: einen Schnitt durch die Stecker-Einheit und Buchsen-Einheit von Fig. 2, wobei die Stecker-Einheit an die Buchsen-Einheit angeschlossen ist, sodass die Strömungsverbindungen hergestellt sind,
- Fig. 7: Die Stecker-Einheit der Vorrichtung zur Bereitstellung von Dialysierflüssigkeit zusammen mit der Buchsen-Einheit der Vorrichtung zum Befüllen der Vorrichtung zur Bereitstellung von Dialysierflüssigkeit in perspektivischer Darstellung,
- Fig. 8A: die Buchsen-Einheit von Fig. 7, die zur Einleitung eines Spülvorgangs vorbereitet ist,
- Fig. 8B: die Buchsen-Einheit von Fig. 7 während des Spülvorgangs,
- Fig. 9: die Stecker-Einheit und Buchsen-Einheit von Fig. 7 in geschnittener Darstellung bevor die Stecker-Einheit und Buchsen-Einheit miteinander verbunden sind,
- Fig. 10: die Stecker-Einheit und Buchsen-Einheit von Fig. 7 in geschnittener Darstellung, wobei die Steckereinheit auf die Buchsen-Einheit lose aufgesetzt ist und
- Fig. 11: einen Schnitt durch die Stecker-Einheit und Buchsen-Einheit von Fig. 7, wobei Stecker-Einheit und Buchsen-Einheit zur Herstellung der Strömungsverbindungen miteinander verbunden sind.

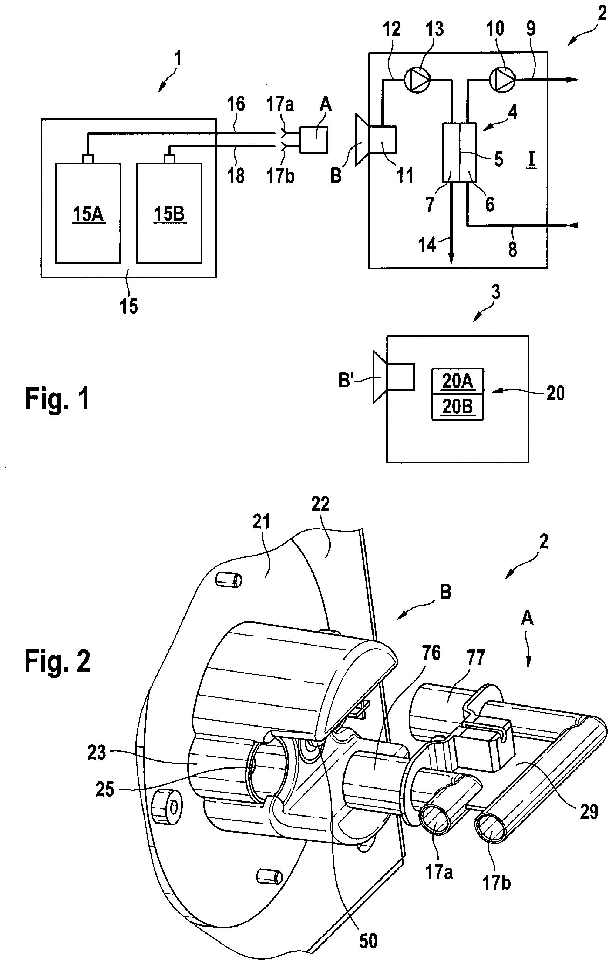

Fig. 1 zeigt in stark vereinfachter schematischer Darstellung eine Vorrichtung 1 zur Bereitstellung einer medizinischen Flüssigkeit, insbesondere Dialysierflüssigkeit, zusammen mit einer Blutbehandlungsvorrichtung 2 und einer Vorrichtung 3 zum Befüllen der Vorrichtung zur Bereitstellung von Dialysierflüssigkeit. Die Blutbehandlungsvorrichtung 2 kann eine extrakorporale Dialysevorrichtung oder eine Vorrichtung zur Peritonealdialyse sein. Bei dem vorliegenden Ausführungsbeispiel ist die Blutbehandlungsvorrichtung 2 eine Dialysevorrichtung, die über einen Dialysator 4 verfügt, der durch eine semipermeable Membran 5 in eine Blutkammer 6 und eine Dialysierflüssigkeitskammer 7 unterteilt ist. Von dem Patienten führt eine Blutzuführleitung 8 zu der Blutkammer 6 des Dialysators 4, während eine Blutrückführleitung 9, in die eine Blutpumpe 10 geschaltet ist, von der Blutkammer 6 zu dem Patienten führt. Die Blutzuführ- und -rückführleitung, 8, 9 bilden zusammen mit der Blutkammer 6 den extrakorporalen Blutkreislauf I der Dialysevorrichtung 2.

Die frische Dialysierflüssigkeit wird aus einem Dialysierflüssigkeitsreservoir 11 über eine Dialysierflüssigkeitszuführleitung 12, in die eine Dialysierflüssigkeitspumpe 13 geschaltet ist, zu der Dialysierflüssigkeitskammer 7 des Dialysators 4 geleitet, während verbrauchte Dialysierflüssigkeit über eine Dialysierflüssigkeitsabführleitung 14 aus der Dialysierflüssigkeitskammer abfließt.

Zur Bereitstellung frischer Dialysierflüssigkeit dient die Vorrichtung 1, die bei dem vorliegenden Ausführungsbeispiel zwei Beutel oder Kanister 15A und 15B aufweist. Beide Beutel oder Kanister 15A, 15B bilden eine Einheit 15, wobei der Beutel 15A vor der Dialysebehandlung mit frischer Dialysierflüssigkeit befüllt und der Beutel 15B leer ist.

Von dem Dialysierflüssigkeitsbeutel 15A führt eine Zulaufleitung 16 zu dem einen Anschluss 17a einer Stecker-Einheit A, während von dem anderen Anschluss 17b der Stecker-Einheit A eine Ablaufleitung 18 zu dem Leerbeutel 15B führt.

Die Stecker-Einheit A wird zur Bereitstellung von Dialysierflüssigkeit vor der Behandlung an eine Buchsen-Einheit B angeschlossen, die an der Blutbehandlungsvorrichtung 2 vorgesehen ist, sodass frische Dialysierflüssigkeit über die Zulaufleitung 16 dem Dialysierflüssigkeitsreservoir 10 zugeführt und verbrauchte Dialysierflüssigkeit über die Ablaufleitung 18 abgeführt werden kann.

Die Vorrichtung 1 zur Bereitstellung von Dialysierflüssigkeit wird an der Vorrichtung 3 mit frischer Dialysierflüssigkeit befüllt. Mit der Vorrichtung 3 zum Befüllen kann die Vorrichtung 2 zum Bereitstellen von Dialysierflüssigkeit auch entleert werden. Zur Aufnahme frischer Dialysierflüssigkeit dient ein Tank 20A und zur Aufnahme verbrauchter Dialysierflüssigkeit dient ein Tank 20B. Die erforderlichen Leitungen und Pumpen werden in der stark schematischen Darstellung nicht gezeigt.

Die Vorrichtung 3 zum Befüllen und Entleeren der Vorrichtung 1 zur Bereitstellung frischer und Aufnahme verbrauchter Dialysierflüssigkeit verfügt über eine Buchsen-Einheit B', an die die Stecker-Einheit A der Vorrichtung 1 zur Bereitstellung von Dialysierflüssigkeit angeschlossen wird. Die Buchsen-Einheit B der Blutbehandlungsvorrichtung 2 und die Buchsen-Einheit B' der Vorrichtung zum Befüllen bzw. Entleeren können identisch oder unterschiedlich ausgebildet sein. Bei dem vorliegenden Ausführungsbeispiel sind die Buchsen-Einheiten B bzw. B' unterschiedlich ausgebildet. Beide Buchsen-Einheiten B bzw. B' sind aber derart ausgebildet, dass sich mit der Stecker-Einheit A der Vorrichtung 1 zur Bereitstellung von Dialysierflüssigkeit eine flüssigkeitsdichte Strömungsverbindung mit beiden Vorrichtungen 2 und 3 in beiden Richtungen für frische und verbrauchte Dialysierflüssigkeit herstellen lässt.

Nachfolgend wird die Stecker-Einheit A der Vorrichtung 1 zur Bereitstellung von Dialysierflüssigkeit zusammen mit der Buchsen-Einheit B der Blutbehandlungsvorrichtung 2 unter Bezugnahme auf die Figuren 2 bis 6 im Einzelnen beschrieben.

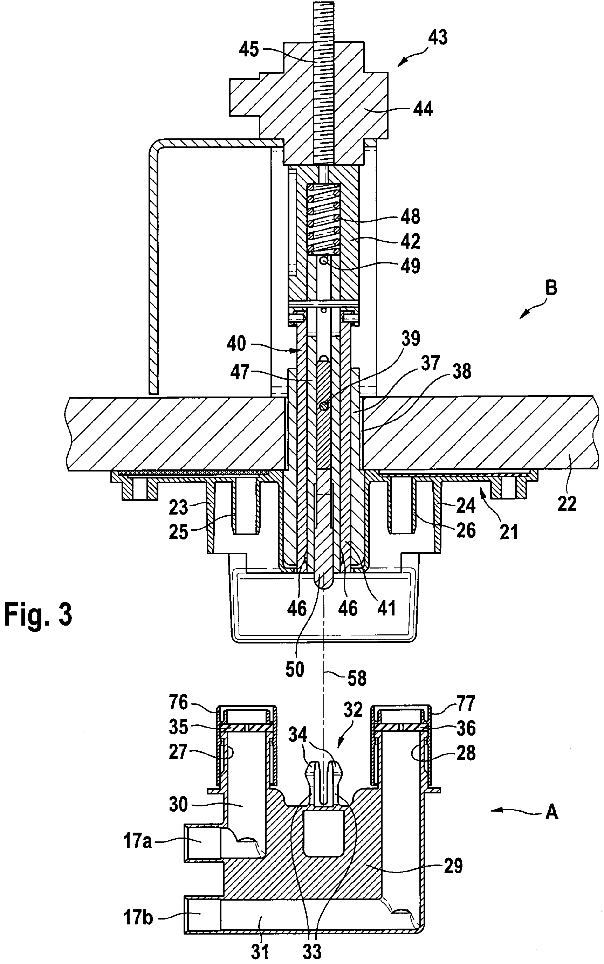

Fig. 2 zeigt die Stecker-Einheit A und die Buchsen-Einheit B in perspektivischer Darstellung, während die Figuren 3 bis 6 die Stecker- und Buchsen-Einheit A, B in geschnittener Darstellung zeigen. Die Buchsen-Einheit B der Blutbehandlungsvorrichtung 2 ist vorzugsweise Teil einer nicht dargestellten Behandlungskassette, die austauschbar ist. Die Buchsen-Einheit B kann aber auch Teil einer nicht austauschbaren Einheit sein. Die Buchsen-Einheit B weist einen äußeren Flanschteil 21 auf, der mit nicht dargestellten Schrauben mit der Gehäusewand 22 der Behandlungskassette bzw. Blutbehandlungsvorrichtung verschraubt ist. Von dem äußeren Flanschteil 21 stehen zwei zylindrische Anschlussteile 23, 24 ab, die in einer gemeinsamen Ebene zu beiden Seiten der zentralen Achse 58 der Buchsen-Einheit angeordnet sind. Die zylindrischen Anschlussteile 23, 24 umschließen jeweils konzentrisch ein Anschlussstück 25 und 26, wobei das Anschlussstück 25 zum Zuführen frischer Dialysierflüssigkeit und das Anschlussstück 26 zum Abführen verbrauchter Dialysierflüssigkeit dient.

Die Stecker-Einheit A der Vorrichtung 1 zur Bereitstellung frischer und zur Aufnahme verbrauchter Dialysierflüssigkeit verfügt über entsprechende Konnektoren 27, 28, die mit den Anschlussstücken 25, 26 flüssigkeitsdicht verbunden werden. Die Stecker-Einheit A weist einen Steckerkörper 29 auf, der die beiden Konnektoren 27, 28 verbindet. Der Steckerkörper 29 weist einen Zulaufkanal 30 auf, der an dem einen Konnektor 27 angeschlossen ist, und weist einen Ablaufkanal 31 auf, der an dem anderen Konnektor 28 angeschlossen ist. An dem Anschluss 17a des Zulaufkanals 30 ist die Zulaufleitung 16 und an dem Anschluss 17b des Ablaufkanals 31 ist die Ablaufleitung 18 der Vorrichtung 1 zur Bereitstellung von frischer bzw. Aufnahme verbrauchter Dialysierflüssigkeit angeschlossen. Zwischen den beiden Konnektoren 27, 28 befindet sich ein Ansatzstück 32, mit dem eine zunächst nur lose Verbindung zwischen der Stecker-Einheit A und der Buchsen-Einheit B hergestellt werden kann. Das Ansatzstück 32 weist mehrere umfangsmäßig verteilt angeordnete Rastelemente 33 auf, die an einem Ende an dem Stecker-Körper 29 angeformt sind. An den Außenseiten der freien Enden der Rastelemente 33 sind Rastnasen 34 ausgebildet. Die Konnektoren 27 und 28 verfügen über Berührungsschutzhülsen 76 und 77, die auf die Konnektoren 27, 28 des Steckerkörpers 29 einrastend aufgesetzt sind. Die Konnektoren 27, 28 werden jeweils von einer Membran 35, 36 verschlossen, die von den Anschlussstücken 25, 26 der Buchsen-Einheit durchdrungen wird.

Im Zentrum des Flanschteils 21 der Buchsen-Einheit B sitzt ein zylindrisches Führungsstück 37, dass sich durch eine Bohrung 38 der Gehäusewand 22 erstreckt. In dem Führungsstück 37 ist ein rohrförmiges Aufnahmestück 40 längsverschiebbar geführt, das einen vorderen Abschnitt 41 und einen hinteren Abschnitt 42 aufweist. Zum Verschieben des rohrförmigen Aufnahmestücks 40 in dem zylindrischen Führungsstück 37 ist eine Antriebseinheit 43 vorgesehen. Die Antriebseinheit 43 ist bei dem vorliegenden Ausführungsbeispiel ein elektromotorischer Spindelantrieb, der einen Linearmotor 44 und eine Spindel 45 aufweist, die mit dem hinteren Abschnitt 42 des Aufnahmestücks 40 verbunden ist. Durch Ein- und Ausfahren der Spindel 45 wird das Aufnahmestück 40 aus dem Führungsstück 37 herausgeschoben oder in das Führungsstück zurückgezogen.

An der Innenseite des vorderen Endes des vorderen Abschnitts 41 des Aufnahmestücks 40 sind mehrere umfangsmäßig verteilt angeordnete Ausnehmungen 46 vorgesehen, die derart ausgebildet sind, dass die Rastnasen 34 der Rastelemente 33 des Ansatzstücks 32 der Stecker-Einheit A in die Ausnehmungen 46 einrasten, wenn das Ansatzstück 32 in das Aufnahmestück 40 eingesetzt wird, was nachfolgend noch im Einzelnen beschrieben wird.

Die Buchsen-Einheit B verfügt über Mittel, mit denen erkannt wird, dass das Ansatzstück 32 in das Aufnahmestück 40 eingesetzt ist. Diese Mittel weisen ein als rohrförmiger Körper ausgebildetes Tastelement 47 auf, das längsverschiebbar in dem rohrförmigen Aufnahmestück 40 geführt ist. Das rohrförmige Tastelement 47 ist mit einer Feder 48 vorgespannt, die in dem hinteren Abschnitt 42 des Aufnahmestücks 40 sitzt. Die Bewegung des Tastelements 47 in Längsrichtung wird von einem nur andeutungsweise dargestellten Anschlagelement 49 begrenzt, das in einem in der vorliegenden Schnittebene nicht sichtbaren Schlitz geführt ist, der in dem Aufnahmestück 40 vorgesehen ist.

Zum Detektieren des Ansatzstücks 32 in dem Aufnahmestück 40 verfügt die Buchsen-Einheit B über Mittel, mit denen erkannt wird, ob das vordere Ende des Tastelements 47 bündig mit dem vorderen Ende des Aufnahmestücks 40 abschließt (Fig. 3) oder entgegen der Vorspannung der Feder 48 in das Aufnahmestück zurückgeschoben ist. Dies ist der Fall, wenn das Ansatzstück 32 in das Aufnahmestück 40 eingesetzt ist.

Die Buchsen-Einheit B weist des Weiteren einen stiftförmigen Körper 50 auf, der unbeweglich innerhalb des rohrförmigen Tastelements 47 angeordnet ist. Der stiftförmige Körper 50 kann beispielsweise mit einem Stift 39 arretiert sein, der sich durch in der Schnittebene nicht dargestellte Schlitze des Tastelements 47 und des Aufnahmestücks 40 in das Führungsstück 37 erstreckt.

Nachfolgend wird im Einzelnen beschrieben, wie die Stecker-Einheit A mit der Buchsen-Einheit B verbunden wird.

Fig. 3 zeigt die Ausgangsposition, in der die Buchsen-Einheit B verriegelt ist. In dieser Position ist die Spindel 45 des Linearmotors 43 eingefahren, wodurch das Aufnahmestück 40 mit dem Tastelement 47 zurückgezogen ist, sodass der stiftförmige Körper 50 aus dem Aufnahmestück vorsteht. Der stiftförmige Körper 50 verhindert also das Einsetzen des Ansatzstücks 32 in das Aufnahmestück 40.

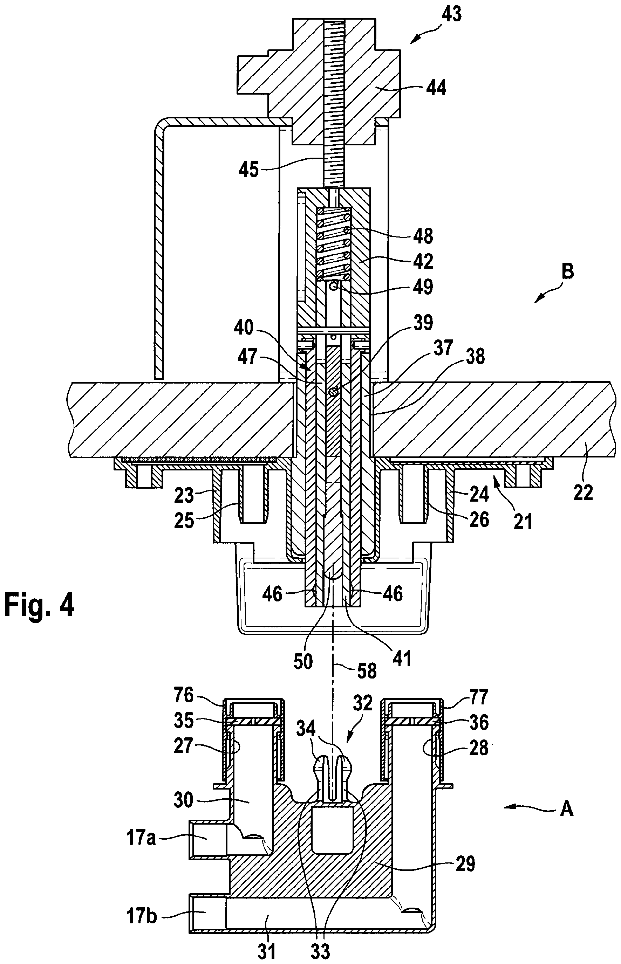

Fig. 4 zeigt die Position des Aufnahmestücks 40 mit dem Tastelement 47, in der die Spindel 45 des Linearmotors 44 ausgefahren ist und das Aufnahmestück 40 mit dem Tastelement 47 aus dem Führungsstück 37 nach außen vorgeschoben ist. In dieser Position ist der stiftförmige Körper 50, der mit dem Führungsstück 37 verbunden ist, soweit zurückgezogen, dass sich das Ansatzstück 32 der Stecker-Einheit A in das Aufnahmestück 40 einsetzen lässt.

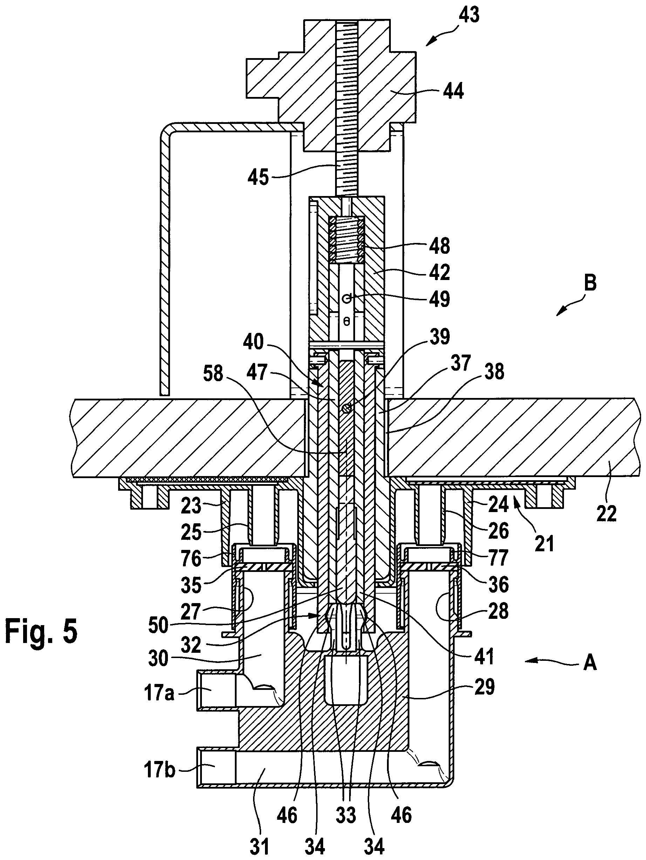

Fig. 5 zeigt das in dem Aufnahmestück 40 der Buchsen-Einheit B sitzende Ansatzstück 32 der Stecker-Einheit A. Beim Einsetzen des Ansatzstücks 32 in das Aufnahmestück 40 rasten die Rastnasen 34 der Rastelemente 33 einschnappend in die Ausnehmungen 46 des Aufnahmestücks 40 ein. Dadurch wird die Stecker-Einheit A lose an der Buchsen-Einheit gehalten. Die Strömungsverbindung ist dabei aber noch nicht hergestellt, da die Anschlussstücke 25, 26 der Buchsen-Einheit B noch nicht mit den Konnektoren 27, 28 der Steckereinheit A verbunden sind. Die Zulauf- und Ablaufkanäle 30, 31 der Stecker-Einheit werden in dieser Position noch von den Membranen 35, 36 flüssigkeitsdicht verschlossen.

Beim Einsetzen des Ansatzstücks 32 in das Aufnahmestück 40 wird das in dem Aufnahmestück längsverschiebbar geführte Tastelement 47 von den Rastelementen 33 des Ansatzstücks 32 entgegen der Federkraft der Feder 48 in das Aufnahmestück 40 zurückgeschoben. Die rückwärtige Stellung des Tastelements 47, die in Fig. 5 dargestellt ist, wird von nicht dargestellten Mitteln, beispielsweise elektrischen Kontakten, die geschlossen werden, oder einer Lichtschranke, erfasst, wodurch die Antriebseinheit 43 in Betrieb gesetzt wird. Der Linearmotor 44 fährt nun die Spindel 45 ein, sodass das Aufnahmestück 40 mit dem Tastelement 47 zurückgezogen wird.

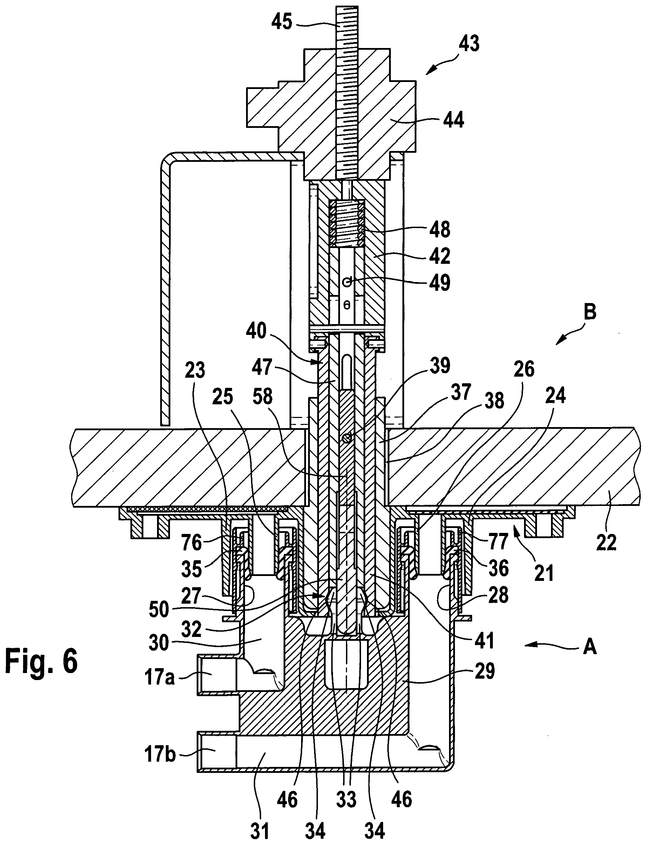

Fig. 6 zeigt die Position des Aufnahmestücks 40, wenn die Spindel 45 des Linearmotors 44 vollständig eingefahren und das Aufnahmestück 40 mit dem Tastelement 37 vollständig zurückgezogen ist. Beim Zurückziehen des Aufnahmestücks 40 wird der stiftförmige Körper 50 in das Ansatzstück 32 vorgeschoben, wodurch die Rastnasen 34 der Rastelemente 33 des Ansatzstücks 32 in den Ausnehmungen 46 des Aufnahmestücks 40 gesichert werden. Dadurch ist das Ansatzstück 32 in dem Aufnahmestück 40 verriegelt. Die Verriegelung des Ansatzstücks 32 in dem Aufnahmestück 40 erfolgt gleichzeitig mit der Relativbewegung der Anschlussstücke 25, 26 und der Konnektoren 27, 28.

In der in Fig. 6 dargestellten Position, in der die Spindel 45 des Linearmotors 44 vollständig eingefahren ist, sind beide Anschlussstücke 25, 26 und Konnektoren 27, 28 flüssigkeitsdicht miteinander verbunden. Die Verriegelung des Ansatzstücks 32 im Aufnahmestück 40 stellt zum einen sicher, dass die zunächst nur lose in die Buchsen-Einheit B eingesetzte Stecker-Einheit A entgegen den auftretenden Kräften auf die Buchsen-Einheit gezogen werden kann und verhindert zum anderen, dass nach Herstellung der Strömungsverbindungen sich die Stecker-Einheit von der Buchsen-Einheit lösen kann. Die flüssigkeitsdichte und nicht lösbare Verbindung zwischen Stecker-Einheit A und Buchsen-Einheit B wird also automatisch nach dem losen Einsetzten der Stecker-Einheit in die Buchsen-Einheit hergestellt.

Die Entriegelung der Stecker-Einheit A von der Buchsen-Einheit B erfolgt in umgekehrter Reihenfolge wie die Verriegelung der Stecker-Einheit an der Buchsen-Einheit. Hierzu wird die Antriebseinheit 43 wieder in Betrieb gesetzt. Dies kann beispielsweise durch Drücken eines Tasters oder dergleichen erfolgen. Wenn die Spindel 45 des Linearmotors 44 wieder ausgefahren wird, schiebt sich das Aufnahmestück 40 mit dem Tastelement 47 wieder über den stiftförmigen Körper 50 nach vorne, wodurch die Verriegelung der Rastverbindung zwischen Ansatzstück 32 und Aufnahmestück 40 aufgehoben wird. Gleichzeitig werden die Anschlussstücke 25, 26 von den Konnektoren 27, 28 getrennt. Damit befindet sich die Stecker-Einheit A wieder in der Ausgangsposition (Fig. 5), in der die Stecker-Einheit noch lose an der Buchsen-Einheit gehalten wird. Dadurch wird verhindert, dass die Stecker-Einheit von der Buchsen-Einheit einfach abfällt.

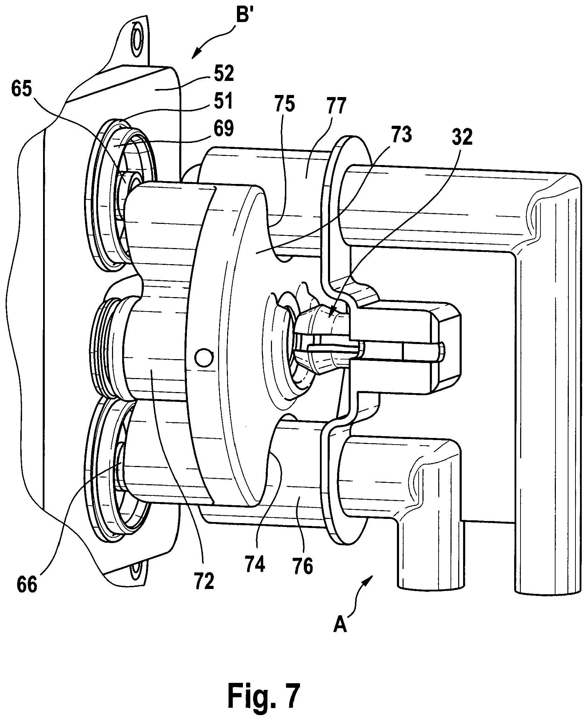

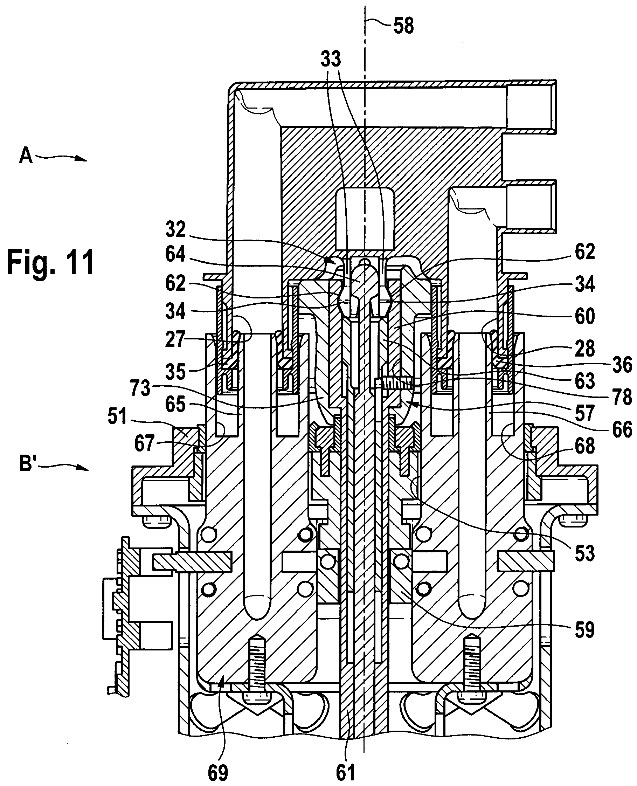

Die Figuren 7-11 zeigen eine alternative Ausführungsform der Buchsen-Einheit B', die an der Vorrichtung 3 zur Befüllung der Vorrichtung 1 zur Bereitstellung der Dialysierflüssigkeit vorgesehen ist. Diese alternative Ausführungsform kann grundsätzlich auch bei der Blutbehandlungsvorrichtung 2 vorgesehen sein. Es ist aber auch möglich, dass die unter Bezugnahme auf die Figuren 2 bis 6 beschriebene Ausführungsform bei der Vorrichtung 3 zur Befüllung vorgesehen ist. Die alternative Ausführungsform der Buchsen-Einheit B' wird nachfolgend im Einzelnen beschrieben.

Fig. 7 zeigt die alternative Ausführungsform der Buchsen-Einheit B' zusammen mit der Stecker-Einheit A in perspektivischer Darstellung. Beide Ausführungsformen der Buchsen-Einheit B und B' können mit derselben Stecker-Einheit A konnektiert werden, sodass sich die Vorrichtung 1 zur Bereitstellung von Dialysierflüssigkeit einerseits an die Vorrichtung 3 zum Befüllen und Entleeren und andererseits an die Blutbehandlungsvorrichtung 2 anschließen lässt.

Die beiden Ausführungsformen der Buchsen-Einheit unterscheiden sich insbesondere dadurch voneinander, dass beim Anschließen der Stecker-Einheit A an die Buchsen-Einheit B zur Herstellung der Strömungsverbindungen die Konnektoren 27, 28 der Stecker-Einheit A automatisch auf die Anschlussstücke 25, 26 der Buchsen-Einheit B gezogen werden (Figuren 2 bis 6), in dem die Stecker-Einheit A bewegt wird, während bei der alternativen Ausführungsform der Buchseneinheit B' die Anschlussstücke der Buchsen-Einheit in die Konnektoren 27, 28 der Stecker-Einheit A eingefahren werden, wobei die Stecker-Einheit A nicht bewegt wird. Darüber hinaus sieht die alternative Ausführungsform der Buchsen-Einheit B' den Verschluss der beiden Anschlussstücke oder die Herstellung einer Strömungsverbindung zwischen beiden Anschlussstücken für einen Spülvorgang vor, ohne dass die Stecker-Einheit A und Buchsen-Einheit B' miteinander verbunden sind.

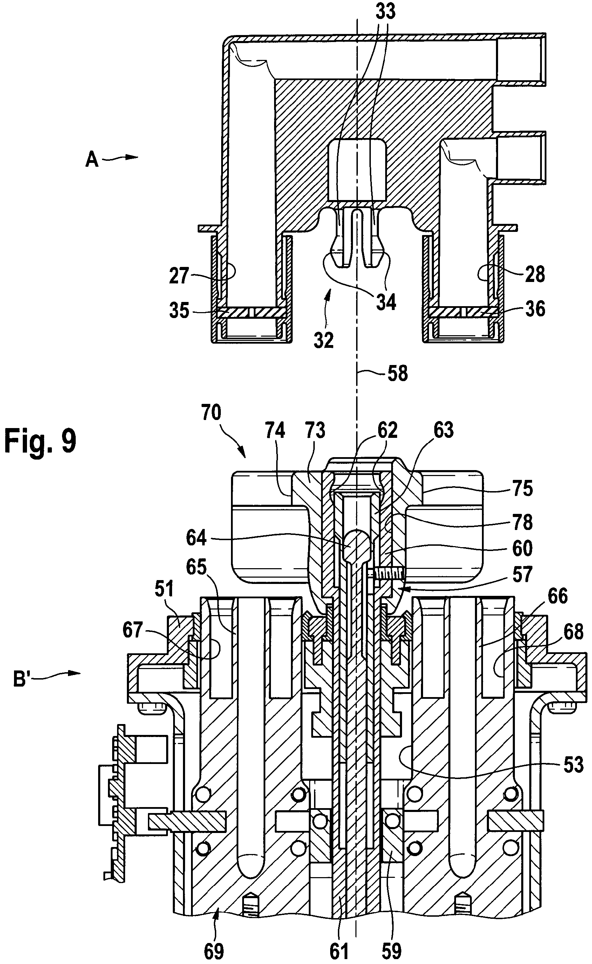

Die Buchsen-Einheit B' weist einen Gehäusekörper 51 auf, der in einer Gehäusewand 52 der Vorrichtung 3 zum Befüllen eingesetzt ist (Fig. 7). Der Gehäusekörper 51 weist eine zentrale Ausnehmung 53 auf, in der das Aufnahmestück 57 für das Ansatzstück 32 der Stecker-Einheit A angeordnet ist (Fig. 9). Im Gegensatz zu der unter Bezugnahme auf die Figuren 2-6 beschriebenen Ausführungsform ist das Aufnahmestück 57 der Buchsen-Einheit B' nicht in Richtung der Längsachse 58 der Buchsen-Einheit B' verschiebbar geführt, sondern um die Längsachse 58 mit einem Lager 59 drehbar gelagert, das in die zentrale Ausnehmung 53 des Gehäusekörpers 51 eingesetzt ist. Das Aufnahmestück 57 wird mit einer nicht dargestellten Antriebseinheit gedreht.

Das Aufnahmestück 57 weist einen sich aus dem Gehäusekörper 51 erstreckenden vorderen Abschnitt 60 und einen sich in den Gehäusekörper 51 erstreckenden hinteren Abschnitt 61 auf, wobei der vordere Abschnitt 60 einen größeren Außen- bzw. Innendurchmesser als der hintere Abschnitt 61 hat. An der Innenseite des vorderen Endes des vorderen Abschnitts 60 des Aufnahmestücks 57 sind die umfangsgemäß verteilt angeordneten Ausnehmungen 62 vorgesehen, in die die Rastnasen 34 der Rastelemente 33 des Ansatzstücks 32 einrasten, wenn die Stecker-Einheit A lose auf die Buchsen-Einheit B' aufgesetzt wird.

In dem rohrförmigen Aufnahmestück 57 ist längsverschiebbar das als rohrförmiger Körper ausgebildete Tastelement 63 geführt, dass mit einer nicht dargestellten Feder vorgespannt ist, sodass beim Einsetzen des Ansatzstücks 32 in das Aufnahmestück 57 das Tastelement 63 entgegen der Federspannung zurückgeschoben wird.

In dem rohrförmigen Tastelement 63 ist der stiftförmige Körper 64 zum Verriegeln des Ansatzstücks 32 in dem Aufnahmestück 57 geführt. Der stiftförmige Körper 64 kann mit einer nicht dargestellten Antriebseinheit in Längsrichtung der Achse 58 vorgeschoben und wieder zurückgezogen werden, um das Ansatzstück 32 in dem Aufnahmestück 57 freizugeben bzw. zu verriegeln.

Bei der alternativen Ausführungsform der Buchseneinheit B' sitzen die Anschlussstücke 65, 66 in zylindrischen Ausnehmungen 67, 68 eines Anschlussteils 69, das in dem Gehäusekörper 51 längsverschiebbar geführt ist, sodass die Anschlussstücke 65, 66 aus dem Gehäusekörper 51 vorgeschoben bzw. in den Gehäusekörper zurückgezogen werden können. Die Antriebseinheit zum Vorschieben bzw. Zurückziehen des Anschlussteils 69 mit den Anschlussstücken 65, 66 ist in den Figuren nicht dargestellt.

Fig. 9 zeigt die Buchsen-Einheit B' in der Position, in der die Stecker-Einheit A lose auf die Buchsen-Einheit B' aufgesetzt werden kann. Der stiftförmige Körper 64 ist in dem Aufnahmestück 57 zurückgezogen, sodass die Rastelemente 33 mit den Rastnasen 34 des Ansatzstücks 32 in das Aufnahmestück 57 mit den Ausnehmungen 62 einrasten können.

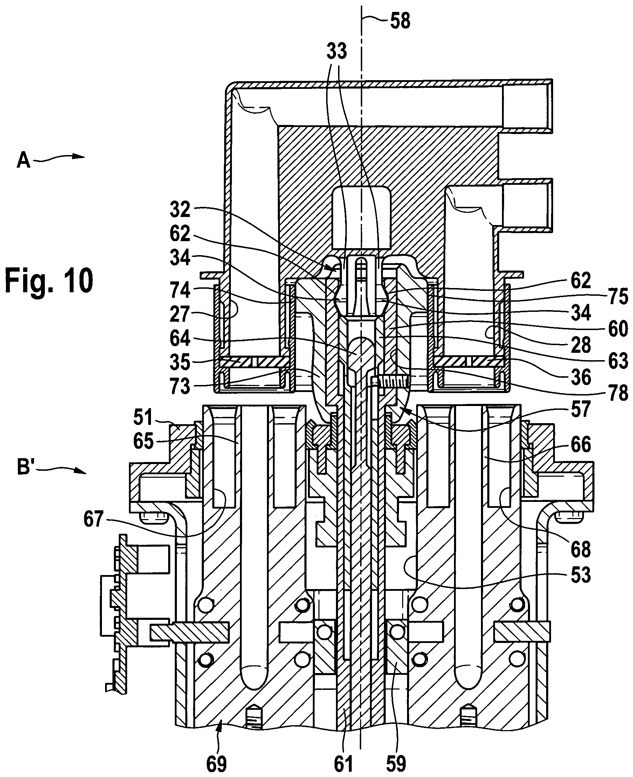

Fig. 10 zeigt die Position, in der die Stecker-Einheit A lose auf die Buchsen-Einheit B' aufgesetzt ist, wobei das Ansatzstück 32 in das Aufnahmestück 57 eingerastet ist. Die Stecker-Einheit A wird dabei nur lose gehalten, ohne dass die Strömungsverbindungen hergestellt sind.

Die Stellung des Tastelements 63 wird wieder überwacht. Da das Tastelement 63 von dem Ansatzstück 32 zurückgeschoben ist, wird erkannt, dass die Stecker-Einheit A lose aufgesetzt ist. Wenn die Stecker-Einheit lose aufgesetzt ist, wird die nicht dargestellte Antriebseinheit in Betrieb gesetzt, wodurch der stiftförmige Körper 64 in dem Aufnahmestück 57 nach vorne geschoben wird. Dadurch wird die zunächst erst lose Verbindung zwischen Ansatzstück 32 und Aufnahmestück 57 verriegelt. Gleichzeitig wird der Anschlussteil 69 mit den beiden Anschlussstücken 65, 66 aus dem Gehäusekörper 51 nach vorne geschoben. Es ist auch möglich, dass der stiftförmige Körper 64 und der Anschlussteil 69 miteinander verbunden sind und von einer Antriebseinheit gemeinsam bewegt werden. Mit dem Verschieben des Anschlussteil 69 mit den beiden Anschlussstücken 65, 66 durchstoßen die Anschlussstücke 65, 66 die Membranen 35, 36 der Stecker-Einheit A, wodurch die flüssigkeitsdichten Verbindungen zwischen den Anschlussstücken und Konnektoren hergestellt wird. Zwar findet wieder eine Relativbewegung zwischen Anschlussstücken 65, 66 und Konnektoren 27, 28 statt. Bei dieser Ausführungsform wird die Stecker-Einheit A aber selbst nicht bewegt. Da die Stecker-Einheit A nach der Verriegelung des Ansatzstücks mit dem Aufnahmestück fest auf der Buchsen-Einheit B' sitzt, können die beim Verbinden von Stecker- und Buchsen-Einheit auftretenden Kräfte aufgenommen werden. Der Anschluss der Stecker-Einheit an die Buchsen-Einheit erfolgt also wieder automatisch.

Das Lösen der Stecker-Einheit A von der Buchsen-Einheit B' erfolgt in umgekehrter Reihenfolge. Hierzu werden der stiftförmige Körper 64 in dem Aufnahmestück 57 und das Anschlussteil 69 mit den Anschlussstücken 65, 66 in dem Gehäusekörper 51 zurückgezogen, wodurch die Verbindung zwischen Ansatzstück 32 und Aufnahmestück 57 entriegelt und die Anschlussstücke 65, 66 aus den Konnektoren 27, 28 gezogen werden. Die Entriegelung kann gleichzeitig mit dem Zurückziehen der Anschlussstücke oder vor dem Zurückziehen der Anschlussstücke erfolgen.

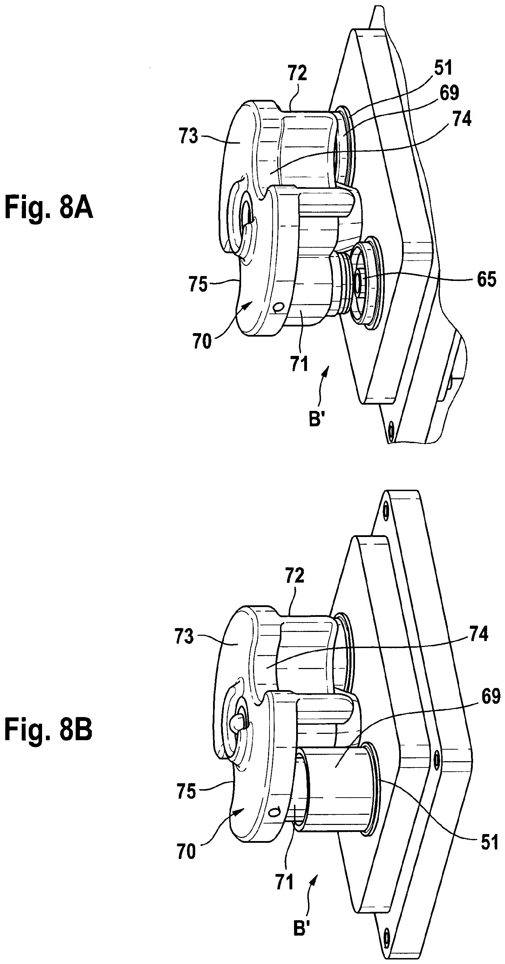

Die alternative Ausführungsform der Buchsen-Einheit B' verfügt über eine Einrichtung 70 zum Verschluss der beiden Anschlussstücke 65, 66 oder zur Herstellung einer Strömungsverbindung zwischen den beiden Anschlussstücken 65, 66, um einen Spülvorgang mit einer Spüllösung durchführen zu können (Fig. 7; Fig. 8A und Fig. 8B). Diese Einrichtung 70 weist zwei Konnektoren 71, 72 auf, die im gleichen Abstand wie die Konnektoren 27, 28 der Stecker-Einheit A zueinander angeordnet sind und die gleiche Ausbildung aufweisen wie die Konnektoren der Stecker-Einheit. Die beiden Konnektoren 71, 72 sind in einem Spülstück 73 an ihrem rückwärtigen Ende verschlossen oder sind in dem Spülstück 73 zur Herstellung einer Strömungsverbindung verbunden.

Das Spülstück 73 weist an den beiden gegenüberliegenden Seiten, an denen die Konnektoren 71, 72 nicht angeordnet sind, halbkreisförmige Einschnitte 74, 75 auf. Das Spülstück 73 mit den Konnektoren 71, 72 ist mit dem vorderen Abschnitt 60 des Aufnahmestücks 57 der Buchsen-Einheit B' verbunden. Dazu weist das Spülstück 73 eine zentrale Ausnehmung 78 auf, durch die sich der vordere Abschnitt 60 des Aufnahmestücks 57 erstreckt (Fig. 9). Da das Aufnahmestück 57 um die Längsachse 58 drehbar gelagert ist, kann durch Drehen des Aufnahmestücks 57 mit der nicht dargestellten Antriebseinheit auch das Spülstück 73 mit den Konnektoren 71, 72 um die Längsachse 58 gedreht werden. Fig. 7 zeigt das Kurzschlussstück 73 mit den Konnektoren 71, 72 in der Position, in der sich die Stecker-Einheit A auf die Buchsen-Einheit B' aufsetzen lässt. In dieser Position befinden sich die halbkreisförmigen Ausnehmungen 74, 75 vor den Anschlussstücken 65, 66 der Buchsen-Einheit B', während die Konnektoren 71, 72 in einer Ebene angeordnet sind, die senkrecht auf der Ebene steht, in der die Anschlussstücke 65, 66 angeordnet sind.

Für die Einleitung des Spülvorgangs wird das Spülstück 73 mit den Konnektoren 71, 72 durch Drehen des Aufnahmestücks 57 von der nicht dargestellten Antriebseinheit um 90° verschwenkt, sodass sich die Konnektoren 71, 72 vor den Anschlusstücken 65, 66 befinden. Damit sind die Anschlussstücke aber noch nicht verschlossen oder eine Strömungsverbindung zwischen den Anschlussstücken ist aber noch nicht hergestellt (Fig. 8A). Daraufhin wird der Anschlussteil 69 mit den Anschlussstücken 65, 66 aus dem Gehäusekörper 51 vorgeschoben, sodass die Konnektoren 65, 66 in die Membranen der Konnektoren 71, 72 eingeschoben werden. Damit wird eine flüssigkeitsdichte Strömungsverbindung zwischen den Anschlussstücken 65, 66 der Buchsen-Einheit B' und den Konnektoren 71, 72 hergestellt, sodass die beiden Anschlussstücke 65, 66 über das Spülstück 73 verschlossen oder kurzgeschlossen sind (Fig. 8B). Nach Beendigung des Spülvorgangs werden die Anschlussstücke 65, 66 wieder zurückgezogen und das Spülstück 73 mit den Konnektoren 71, 72 in die Ausgangsposition (Fig.7) zurückgedreht.

Die besondere Ausbildung der Einrichtung 70 zum Verschluss oder zur Herstellung der Strömungsverbindung stellt einen Bestandteil der Buchsen-Einheit B' dar. Ein separater Stecker oder dergleichen ist daher nicht erforderlich. Die Buchsen-Einheit B' erlaubt eine vollautomatische Steuerung sowohl des Anschlusses der Stecker-Einheit A an die Buchsen-Einheit B' als auch der Einleitung des Spülvorgangs, sodass die Handhabung insgesamt vereinfacht wird. Da das Einsetzen der Stecker-Einheit in die Buchsen-Einheit erkannt wird, kann der Befüllvorgang oder Entleervorgang automatisch eingeleitet werden. Nach dem Befüllen oder Entleeren kann die Stecker-Einheit automatisch freigegeben werden. Das gleiche gilt auch für den Spülvorgang. Auch beim Anschluss der Vorrichtung 1 zur Bereitstellung von Dialysierflüssigkeit an die Dialysevorrichtung 2 kann die Befüllung des Flüssigkeitsreservoirs 10 mit dem Einsetzen der Stecker-Einheit A in die Buchsen-Einheit B der Dialysevorrichtung automatisch gestartet werden.

## Patentansprüche

1. Medizinische Vorrichtung mit einer Buchsen-Einheit (B, B') zum Anschluss einer Stecker-Einheit einer Vorrichtung zur Bereitstellung von medizinischen Flüssigkeiten, wobei
die Buchsen-Einheit (B, B') mindestens ein Anschlussstück (25, 26; 65, 66) zum Anschluss mindestens eines Konnektors der Stecker-Einheit aufweist, so dass beim Anschluss des Konnektors an das Anschlussstück eine Strömungsverbindung zum Zuführen frischer oder Abführen verbrauchter Behandlungsflüssigkeit herstellbar ist, und
die Buchsen-Einheit (B) Mittel (40, 50; 57, 64) zum Verbinden der Stecker-Einheit und der Buchsen-Einheit aufweist, die derart ausgebildet sind, dass bei der Ausführung einer Relativbewegung zwischen dem mindestens einen Anschlussstück der Buchsen-Einheit und dem mindestens einen Konnektor der Stecker-Einheit die Stecker-Einheit und Buchsen-Einheit miteinander verbunden sind,
**dadurch gekennzeichnet, dass**
die Mittel (40, 50; 57, 64) zum Verbinden der Stecker-Einheit und der Buchsen-Einheit ein in Längsrichtung bewegbares Aufnahmestück (40, 57) für ein Ansatzstück der Stecker-Einheit aufweisen, und
die Mittel (40, 43; 57) zum Bewegen des mindestens einen Anschlussstücks der Buchsen-Einheit oder des mindestens einen Konnektors der Stecker-Einheit eine Antriebseinheit (43) zum Bewegen des Aufnahmestücks (40, 57) aufweisen.

2. Medizinische Vorrichtung nach Anspruch 1, **dadurch gekennzeichnet, dass** die Mittel (40, 43; 57) zum Bewegen des mindestens einen Anschlussstücks (25, 26; 65, 66) der Buchsen-Einheit (B, B') oder des mindestens einen Konnektors der Stecker-Einheit und die Mittel (40, 50; 57, 64) zum Verbinden der Stecker-Einheit und der Buchsen-Einheit derart zusammenwirken, dass die Verbindung der Stecker-Einheit und der Buchsen-Einheit gleichzeitig mit der Ausführung der Relativbewegung zwischen dem mindestens einen Anschlussstück der Buchsen-Einheit und dem mindestens einen Konnektor der Stecker-Einheit erfolgt.

3. Medizinische Vorrichtung nach Anspruch 1 oder 2, **dadurch gekennzeichnet, dass** das mindestens eine Anschlussstück (25, 26) der Buchsen-Einheit (B) unbeweglich an der Buchsen-Einheit angeordnet ist, wobei die Mittel (40, 43) zum Bewegen des mindestens einen Anschlussstücks (25, 26) der Buchsen-Einheit (B) oder des mindestens einen Konnektors der Stecker-Einheit derart ausgebildet sind, dass die Stecker-Einheit auf die Buchsen-Einheit zu- oder von der Buchsen-Einheit weg bewegbar ist.

4. Medizinische Vorrichtung nach einem der Ansprüche 1 bis 3, **dadurch gekennzeichnet, dass** das Aufnahmestück (40) der Buchsen-Einheit (B) als ein in Längsrichtung verschiebbarer rohrförmigen Körper (40) ausgebildet ist, in den das Ansatzstück der Stecker-Einheit einsetzbar ist.

5. Medizinische Vorrichtung nach einem der Ansprüche 1 bis 4, **dadurch gekennzeichnet, dass** das Aufnahmestück (40; 57) der Buchsen-Einheit (B, B') Ausnehmungen (46; 62) zur Aufnahme von Rastnasen des Ansatzstücks der Stecker-Einheit aufweist.

6. Medizinische Vorrichtung nach einem der Ansprüche 1 bis 5, **dadurch gekennzeichnet, dass** die Mittel (40, 50; 57, 64) zum Verbinden der Stecker-Einheit und der Buchsen-Einheit einen stiftförmigen Körper (50; 64) aufweisen, der in das Ansatzstück der Stecker-Einheit einführbar ist, so dass zur Herstellung einer Verbindung zwischen der Stecker-Einheit und der Buchsen-Einheit das Ansatzstück der Stecker-Einheit aufspreizbar ist.

7. Medizinische Vorrichtung nach Anspruch 6, **dadurch gekennzeichnet, dass** der stiftförmige Körper (50) unbeweglich in der Buchsen-Einheit (B) angeordnet ist.

8. Medizinische Vorrichtung nach Anspruch 7, **dadurch gekennzeichnet, dass** der stiftförmige Körper (50) in dem rohrförmigen Körper (40) angeordnet ist.

9. Medizinische Vorrichtung nach einem der Ansprüche 1 bis 8, **dadurch gekennzeichnet, dass** die Mittel (40, 50; 57, 64) zum Verbinden der Stecker-Einheit und der Buchsen-Einheit Mittel (47, 63) zum Detektieren des Ansatzstücks der Stecker-Einheit in dem Aufnahmestück (40, 57) der Buchsen-Einheit aufweist.

10. Medizinische Vorrichtung nach Anspruch 9, **dadurch gekennzeichnet, dass** die Mittel (47, 63) zum Detektieren des Ansatzstücks der Stecker-Einheit ein federnd vorgespanntes Tastelement (47, 63) aufweisen, das derart in der Buchsen-Einheit angeordnet ist, dass das Tastelement beim Einführen des Ansatzstücks der Stecker-Einheit in das Aufnahmestück der Buchsen-Einheit entgegen der Federspannung verschiebbar ist.

11. Medizinische Vorrichtung nach Anspruch 10, **dadurch gekennzeichnet, dass** das Tastelement (47, 63) als ein rohrförmiger Körper (47, 63) ausgebildet ist.

12. Medizinische Vorrichtung nach Anspruch 11, **dadurch gekennzeichnet, dass** das Aufnahmestück (40; 57), das der rohrförmige Körper (47; 63) und der stiftförmige Körper (50; 64) konzentrisch angeordnet sind.

13. Medizinische Vorrichtung nach einem der Ansprüche 1 bis 12, **dadurch gekennzeichnet, dass** die Buchsen-Einheit (B) ein erstes Anschlussstück zum Anschluss eines ersten Konnektors (25, 65) der Stecker-Einheit und ein zweites Anschlussstück (26, 66) zum Anschluss eines zweiten Konnektors der Stecker-Einheit aufweist, so dass eine erste Strömungsverbindung zum Zuführen frischer Behandlungsflüssigkeit und eine zweite Strömungsverbindung zum Abführen verbrauchter Behandlungsflüssigkeit herstellbar ist.

14. Medizinische Vorrichtung nach einem der Ansprüche 1 bis 13, **dadurch gekennzeichnet, dass** die medizinische Vorrichtung eine Blutbehandlungsvorrichtung (2), insbesondere eine extrakorporale Dialysevorrichtung oder eine Vorrichtung zur Peritionealdialyse ist.

15. Medizinische Vorrichtung nach einem der Ansprüche 1 bis 14, **dadurch gekennzeichnet, dass** die medizinische Vorrichtung eine Vorrichtung (3) zum Befüllen einer Vorrichtung zur Bereitstellung von medizinischen Flüssigkeiten für eine Blutbehandlungsvorrichtung, insbesondere eine extrakorporale Dialysevorrichtung oder eine Vorrichtung zur Peritionealdialyse ist.

16. Anordnung mit einer medizinischen Vorrichtung nach einem der Ansprüche 1 bis 15 und einer Vorrichtung zur Bereitstellung von medizinischen Flüssigkeiten, wobei die Vorrichtung (1) zur Bereitstellung von medizinischen Flüssigkeiten eine Stecker-Einheit (A) zum Anschluss an die Buchsen-Einheit der medizinischen Vorrichtung aufweist, wobei die Stecker-Einheit (A) mindestens einen Konnektor (27, 28) zum Anschluss an mindestens ein Anschlussstück der Buchsen-Einheit aufweist, so dass mindestens eine Strömungsverbindung zum Zuführen frischer oder Abführen verbrauchter Behandlungsflüssigkeit herstellbar ist,
**dadurch gekennzeichnet, dass** die Stecker-Einheit (A) ein Ansatzstück (32) aufweist, das in ein Aufnahmestück der Buchsen-Einheit einsetzbar ist, wobei das Ansatzstück (32) der Stecker-Einheit (A) derart ausgebildet ist, dass das Ansatzstück in dem Aufnahmestück der Stecker-Einheit verriegelbar ist.

17. Vorrichtung nach Anspruch 16, **dadurch gekennzeichnet, dass** das Ansatzstück (32) der Stecker-Einheit (A) mit Rastnasen (34) versehene Rastelemente (33) aufweist.

18. Vorrichtung nach Anspruch 17, **dadurch gekennzeichnet, dass** die Rastelemente (33) umfangsmäßig verteilt angeordnet sind, wobei die Rastnasen (34) an den Außenseiten der freien Enden der Rastelemente (33) ausgebildet sind.

19. Vorrichtung nach einem der Ansprüche 16 bis 18, **dadurch gekennzeichnet, dass** die Stecker-Einheit (A) einen ersten Konnektor (27) zum Anschluss an ein erstes Anschlussstück der Buchsen-Einheit und einen zweiten Konnektor (28) zum Anschluss an ein zweites Anschlussstück der Buchsen-Einheit aufweist, so dass mindestens eine erste Strömungsverbindung zum Zuführen frischer Behandlungsflüssigkeit und eine zweite Strömungsverbindung zum Abführen verbrauchter Behandlungsflüssigkeit herstellbar ist.

## Claims

1. Medical apparatus having a socket unit (B, B') for the connection of a plug unit of a device for supplying medical fluids,
the socket unit (B, B') having at least one connecting piece (25, 26; 65, 66) for connecting at least one connector of the plug unit, thus enabling a flow-permitting connection to allow fresh treatment fluid to be fed in or used treatment fluid to be fed out to be made when the connector is connected to the connecting piece,
the socket unit (B) having means (40, 50; 57, 64) for connecting the plug unit and the socket unit which are so designed that the plug unit and socket unit are connected together when a relative movement occurs between the at least one connecting piece of the socket unit and the at least one connector of the plug unit,
**characterised in that**,
the means (40, 50; 57, 64) for connecting the plug unit and the socket unit have a receiving piece (40, 57) which is movable in the longitudinal direction for a projecting piece of the plug unit, and
the means (40, 43; 57) for moving the at least one connecting piece of the socket unit or the at least one connector of the plug unit have a drive unit (43) for moving the receiving piece (40, 57).

2. Medical apparatus according to claim 1, **characterised in that** the means (40, 43; 57) for moving the at least one connecting piece (25, 26; 65, 66) of the socket unit (B. B') or the at least one connector of the plug unit, and the means (40, 50; 57, 64) for connecting the plug unit and the socket unit cooperate in such a way that the connection of the plug unit and socket unit takes place simultaneously with the occurrence of the relative movement between the at least one connecting piece of the socket unit and the at least one connector of the plug unit.

3. Medical apparatus according to claim 1 or 2, **characterised in that** the at least one connecting piece (25, 26) of the socket unit (B) is arranged to be immovable on the socket unit, the means (40, 43) for moving the at least one connecting piece (25, 26) of the socket unit (B) or the at least one connector of the plug unit being so designed that the plug unit can be moved towards the socket unit or away from the socket unit.

4. Medical apparatus according to one of claims 1 to 3, **characterised in that** the receiving piece (40) of the socket unit (B) takes the form of a tubular body (40) displaceable in the longitudinal direction in which the projecting piece of the plug unit can be inserted.

5. Medical apparatus according to one of claims 1 to 4, **characterised in that** the receiving piece (40; 57) of the socket unit (B, B') has recesses (46; 62) to receive latching noses of the projecting piece of the plug unit.

6. Medical apparatus according to one of claims 1 to 5, **characterised in that** the means (40, 50; 57, 64) for connecting the plug unit and the socket unit have a body (50; 64) in pin form which can be introduced into the projecting piece of the plug unit, thus enabling the projecting piece of the plug unit to be splayed apart to make a connection between the plug unit and socket unit.

7. Medical apparatus according to claim 6, **characterised in that** the body (50) in pin form is arranged to be immobile in the socket unit (B).

8. Medical apparatus according to claim 7, **characterised in that** the body (50) in pin form is arranged in the tubular body (40).

9. Medical apparatus according to one of claims 1 to 8, **characterised in that** the means (40, 50; 57, 64) for connecting the plug unit and socket unit have means (47, 63) for detecting the projecting piece of the plug unit in the receiving piece (40, 57) of the socket unit.

10. Medical apparatus according to claim 9, **characterised in that** the means (47, 63) for detecting the projecting piece of the plug unit have a sensing member (47, 63) which is resiliently pre-loaded and which is so arranged in the socket unit that, when the projecting piece of the plug unit is introduced into the receiving piece of the socket unit, the said sensing member is displaceable in opposition to the resilient loading.

11. Medical apparatus according to claim 10, **characterised in that** the sensing member (47, 63) takes the form of a tubular body (47, 63).

12. Medical apparatus according to claim 11, **characterised in that** the receiving piece (40, 57), the tubular body (47, 63) and the body (50, 64) in pin form are concentrically arranged.

13. Medical apparatus according to one of claims 1 to 12, **characterised in that** the socket unit (B) has a first connecting piece for connection to a first connector (25, 65) of the plug unit and a second connecting piece (26, 66) for connection to a second connector of the plug unit, thus enabling a first flow-permitting connection to be made for feeding in fresh treatment fluid and a second flow-permitting connection to be made for feeding out used treatment fluid.

14. Medical apparatus according to one of claims 1 to 13, **characterised in that** the medical apparatus is a blood treatment apparatus (2) and in particular an extra-corporeal dialysis apparatus or an apparatus for peritoneal dialysis.

15. Medical apparatus according to one of claims 1 to 14, **characterised in that** the medical apparatus is an apparatus (3) for filling a device for supplying medical fluids for a blood treatment apparatus and in particular an extra-corporeal dialysis apparatus or an apparatus for peritoneal dialysis.

16. Arrangement comprising a medical apparatus according to one of claims 1 to 15 and a device for supplying medical fluids, the device (1) for supplying medical fluids having a plug unit (A) for connection to the socket unit of the medical apparatus, the plug unit (A) having at least one connector (27, 28) for connection to at least one connecting piece of the socket unit, thus enabling at least one flow-permitting connection to be made to allow fresh treatment fluid to be fed in or used treatment fluid to be fed out,
**characterised in that** the plug unit (A) has a projecting piece (32) which can be inserted in a receiving piece of the socket unit, the projecting piece (32) of the plug unit (A) being so designed that the projecting piece can be locked in the receiving piece of the plug unit.

17. Device according to claim 16, **characterised in that** the projecting piece (32) of the plug unit (A) has latching members (33) which are provided with latching noses (34).

18. Device according to claim 17, **characterised in that** the latching members (33) are arranged to be circumferentially distributed, the latching noses (34) being formed on the outer sides of the free ends of the latching members (33).

19. Device according to one of claims 16 to 18, **characterised in that** the plug unit (A) has a first connector (27) for connection to a first connecting piece of the socket unit and a second connector (28) for connection to a second connecting piece of the socket unit, thus enabling at least one first flow-permitting connection to be made for feeding in fresh treatment fluid and one second flow-permitting connection to be made for feeding out used treatment fluid.

## Revendications

1. Dispositif médical avec une unité de prise femelle (B, B') servant au raccordement d'une unité de prise mâle d'un dispositif servant à fournir des liquides médicaux, dans lequel
l'unité de prise femelle (B, B') présente au moins une pièce de raccordement (25, 26 ; 65, 66) servant à raccorder au moins un connecteur de l'unité de prise mâle, de sorte que lors du raccordement du connecteur à la pièce de raccordement, une liaison fluidique servant à amener du liquide de traitement frais ou à évacuer du liquide de traitement usagé peut être établie, et
l'unité de prise femelle (B) présente des moyens (40, 50 ; 57, 64) servant à relier l'unité de prise mâle et l'unité de prise femelle, qui sont réalisés de telle manière que lors de l'exécution d'un déplacement relatif entre l'au moins une pièce de raccordement de l'unité de prise femelle et l'au moins un connecteur de l'unité de prise mâle, l'unité de prise mâle et l'unité de prise femelle sont reliées l'une à l'autre,
**caractérisé en ce que**
les moyens (40, 50 ; 57, 64) servant à relier l'unité de prise mâle et l'unité de prise femelle présentent une pièce de logement (40, 57) pouvant être déplacée dans le sens longitudinal pour un embout de l'unité de prise mâle, et
les moyens (40, 43 ; 57) servant à déplacer l'au moins une pièce de raccordement de l'unité de prise femelle ou l'au moins un connecteur de l'unité de prise mâle présentent une unité d'entraînement (43) servant à déplacer la pièce de logement (40, 57).

2. Dispositif médical selon la revendication 1, **caractérisé en ce que** les moyens (40, 43 ; 57) servant à déplacer l'au moins une pièce de raccordement (25, 26 ; 65, 66) de l'unité de prise femelle (B, B') ou l'au moins un connecteur de l'unité de prise mâle et les moyens (40, 50 ; 57, 64) servant à relier l'unité de prise mâle et l'unité de prise femelle coopèrent de telle manière que la liaison de l'unité de prise mâle et de l'unité de prise femelle est effectuée de manière simultanée avec l'exécution du déplacement relatif entre l'au moins une pièce de raccordement de l'unité de prise femelle et l'au moins un connecteur de l'unité de prise mâle.

3. Dispositif médical selon la revendication 1 ou 2, **caractérisé en ce que** l'au moins une pièce de raccordement (25, 26) de l'unité de prise femelle (B) est disposée de manière immobile au niveau de l'unité de prise femelle, dans lequel les moyens (40, 43) servant à déplacer l'au moins une pièce de raccordement (25, 26) de l'unité de prise femelle (B) ou l'au moins un connecteur de l'unité de prise mâle sont réalisés de telle manière que l'unité de prise mâle peut être déplacée vers l'unité de prise femelle ou à l'opposé de l'unité de prise femelle.

4. Dispositif médical selon l'une quelconque des revendications 1 à 3, **caractérisé en ce que** la pièce de logement (40) de l'unité de prise femelle (B) est réalisée en tant que corps tubulaire (40) pouvant être coulissé dans le sens longitudinal, dans lequel l'embout de l'unité de prise mâle peut être inséré.

5. Dispositif médical selon l'une quelconque des revendications 1 à 4, **caractérisé en ce que** la pièce de logement (40 ; 57) de l'unité de prise femelle (B, B') présente des évidements (46 ; 62) servant à loger des ergots d'enclenchement de l'embout de l'unité de prise mâle.

6. Dispositif médical selon l'une quelconque des revendications 1 à 5, **caractérisé en ce que** les moyens (40, 50 ; 57, 64) servant à relier l'unité de prise mâle et l'unité de prise femelle présentent un corps en forme de tige (50 ; 64), qui peut être introduit dans l'embout de l'unité de prise mâle de sorte que pour établir une liaison entre l'unité de prise mâle et l'unité de prise femelle, l'embout de l'unité de prise mâle peut être expansé.

7. Dispositif médical selon la revendication 6, **caractérisé en ce que** le corps en forme de tige (50) est disposé de manière immobile dans l'unité de prise femelle (B).

8. Dispositif médical selon la revendication 7, **caractérisé en ce que** le corps en forme de tige (50) est disposé dans le corps tubulaire (40).

9. Dispositif médical selon l'une quelconque des revendications 1 à 8, **caractérisé en ce que** les moyens (40, 50 ; 57, 64) servant à relier l'unité de prise mâle et l'unité de prise femelle présentent des moyens (47, 63) servant à détecter l'embout de l'unité de prise mâle dans la pièce de logement (40, 57) de l'unité de prise femelle.

10. Dispositif médical selon la revendication 9, **caractérisé en ce que** les moyens (47, 63) servant à détecter l'embout de l'unité de prise mâle présentent un élément de sondage (47, 63) précontraint sur ressorts, qui est disposé de telle manière dans l'unité de prise femelle que l'élément de sondage peut être coulissé lors de l'introduction de l'embout de l'unité de prise mâle dans la pièce de logement de l'unité de prise femelle à l'encontre de la tension de ressort.

11. Dispositif médical selon la revendication 10, **caractérisé en ce que** l'élément de sondage (47, 63) est réalisé en tant que corps tubulaire (47, 63).

12. Dispositif médical selon la revendication 11, **caractérisé en ce que** la pièce de logement (40 ; 57), le corps tubulaire (47 ; 63) et le corps en forme de tige (50 ; 64) sont disposés de manière concentrique.

13. Dispositif médical selon l'une quelconque des revendications 1 à 12, **caractérisé en ce que** l'unité de prise femelle (B) présente une première pièce de raccordement servant au raccordement d'un premier connecteur (25, 65) de l'unité de prise mâle et une deuxième pièce de raccordement (26, 66) servant au raccordement d'un deuxième connecteur de l'unité de prise mâle, de sorte qu'une première liaison fluidique peut être établie pour amener un liquide de traitement frais et une deuxième liaison fluidique peut être établie pour évacuer du liquide de traitement usagé.

14. Dispositif médical selon l'une quelconque des revendications 1 à 13, **caractérisé en ce que** le dispositif médical est un dispositif de traitement du sang (2), en particulier un dispositif de dialyse extracorporelle ou un dispositif de dialyse péritonéale.

15. Dispositif médical selon l'une quelconque des revendications 1 à 14, **caractérisé en ce que** le dispositif médical est un dispositif (3) servant à remplir un dispositif servant à fournir des liquides médicaux pour un dispositif de traitement du sang, en particulier un dispositif de dialyse extracorporelle ou un dispositif de dialyse péritonéale.

16. Ensemble avec un dispositif médical selon l'une quelconque des revendications 1 à 15 et un dispositif servant à fournir des liquides médicaux, dans lequel le dispositif (1) servant à fournir des liquides médicaux présente une unité de prise mâle (A) destinée à être raccordée à l'unité de prise femelle du dispositif médical, dans lequel l'unité de prise mâle (A) présente au moins un connecteur (27, 28) destiné à être raccordé à au moins une pièce de raccordement de l'unité de prise femelle de sorte qu'au moins une liaison fluidique servant à amener un liquide de traitement frais ou à évacuer un liquide de traitement usagé peut être établie,
**caractérisé en ce que** l'unité de prise mâle (A) présente un embout (32), qui peut être inséré dans une pièce de logement de l'unité de prise femelle, dans lequel l'embout (32) de l'unité de prise mâle (A) est réalisé de telle manière que l'embout peut être verrouillé dans la pièce de logement de l'unité de prise mâle.

17. Dispositif selon la revendication 16, **caractérisé en ce que** l'embout (32) de l'unité de prise mâle (A) présente des éléments d'enclenchement (33) pourvus d'ergots d'enclenchement (34).

18. Dispositif selon la revendication 17, **caractérisé en ce que** les éléments d'enclenchement (33) sont disposés de manière répartie en périphérie, dans lequel les ergots d'enclenchement (34) sont réalisés au niveau des côtés extérieurs des extrémités libres des éléments d'enclenchement (33).

19. Dispositif selon l'une quelconque des revendications 16 à 18, **caractérisé en ce que** l'unité de prise mâle (A) présente un premier connecteur (27) destiné à être raccordé à une première pièce de raccordement de l'unité de prise femelle et un deuxième connecteur (28) destiné à être raccordé à une deuxième pièce de raccordement de l'unité de prise femelle, de sorte qu'au moins une première liaison fluidique servant à amener du liquide de traitement frais et une deuxième liaison fluidique servant à évacuer du liquide de traitement usagé peuvent être établies.
